# DEMANDE DE BREVET EUROPEEN

(11) **EP 2 095 811 A1**
(43) Date de publication de la demande: **02.09.2009**
(21) Numéro de dépôt: 08368006.6
(22) Date de dépôt: 27.02.2008
(51) Int. Cl.: A61K 8/97, A61K 8/98, A61Q 7/00, A61Q 19/02, A61Q 19/08, A61K 9/06, A61K 36/25, A61K 36/48, A61K 36/8998

(54) **Onguent pour arrêter la chute des cheveux, favoriser leur repousse ainsi que la disparition des taches de vieillesse et des rides, supprimer l'inflammation dans les phlébites et les varices**

(71) Demandeur: Candia, Louis, 06100 Nice (FR)
(72) Inventeur: Candia, Louis, 06100 Nice (FR)

(57) **Abrégé**

Association synergique sous forme d'onguent pour arrêter la chute des cheveux, favoriser leur repousse ainsi que la disparition des tâches de vieillesse et des rides, supprimer l'inflammation dans les phlébites et les varices.

L'invention a pour objet un onguent pour arrêter la chute des cheveux, favoriser leur repousse ainsi que la disparition des tâches de vieillesse et des rides, supprimer l'inflammation dans les phlébites et les varices.

Il est constitué d'une combinaison des substances suivantes:
miel d'acacia,
lierre,
orge
mélangés à de l'alcool à 90°
et d'eau et d'huile d'olive, de tournesol ou de maïs.

## Description

L'invention a pour objet une association synergique de type onguent pour une posologie typique destinée à la réactivation du circuit veineux superficiel et à l'élimination des troubles liés à son inefficacité en particulier la formation de cellulite. La formule décrite ci-après se base sur la combinaison synergétique de trois principes particuliers : le lierre, le miel d'acacia et l'orge.

L'onguent selon l'invention est particulièrement efficace pour arrêter la chute des cheveux et favoriser leur repousse, pour la disparition progressive sur le visage des tâches de vieillesse et des rides; enfin, pour supprimer l'inflammation dans les phlébites et les varices.

L'état de la technique peut être défini par les brevets suivants:
- IT 01317068 : l'invention a pour objet une crème pour la peau basée sur la combinaison synergétique de trois principes actifs particuliers dont l'action active du point de vue pharmacologique sur les ramifications veineuses et artérielles du cirque périphérique est ultérieurement accrue soit par la présence d'antioxydants et d'inhibiteurs, soit par une concentration élevée de lécithine de soja (3%) qui leur permet une rapide pénétration au niveau dermique, où les trois principes actifs doivent exercer leur fonction. Ladite crème trouve son application thérapeutique dans le traitement de la cellulite, des troubles dus à l'inefficacité du circuit veineux superficiel, agit contre la sensation de lourdeur, de faiblesse et de fatigue des articulations, les crampes, les tuméfactions et les oedèmes d'origine inflammatoire, les ecchymoses, les hématomes et les engelures.
- FR 2598912 : L'invention a pour objet une composition capillaire pour le traitement des matières kératiniques et en particulier pour la repousse des cheveux, la croissance, l'irrigation du cuir chevelu et la prévention de la chute des cheveux, caractérisée en ce qu'elle comporte les éléments suivants : pétrole, eau, souffre, teinture d'iode, parfum et citron.
- FR 2490960 : L'invention a pour objet un produit assurant la repouse des cheveux et la revitalisation des cheveux blancs comprenant deux lotions que l'on passe l'une après l'autre : en 1^{er}, feuilles de romarin macérées dans de l'alcoolat de romarin ; en 2^{nd}, feuilles de romarin macérées dans de l'huile de ricin.

L'onguent, objet de l'invention, est constitué d'une combinaison des substances suivantes: lierre, miel d'acacia, orge, huile d'olive, huile de tournesol, huile de maïs, alcool à 90° et eau.

Le lierre est une feuille qui, du point de vue cosmétique, de nos jours, est utilisée quasi exclusivement en préparations anti-cellulite qui détendent la peau contractée, calment les douleurs et réduisent d'une manière significative la lourdeur due aux nodules typiques de celle-ci.

Le miel d'acacia associé au lierre et à l'orge apporte à l'organisme les vitamines du groupe B essentiellement.

L'orge qui contient la vitamine B redonne sa vitalité à la peau.

Il est communément admis que pour 100 g de miel de fleurs d'acacia, la valeur énergétique est de 1404 kj (330 Kcal) et contient 0.1 g de protéines, 82.5 g de carboidrates et 0 g de matières grasses.

La composition dudit onguent est la suivante :
Miel d'acacia
Lierre
Orge
le tout mélangé à de l'huile, de l'alcool à 90° et à de l'eau.

## Revendications

1. Association synergique **caractérisée en ce qu'**elle contient du lierre, du miel d'acacia, de l'orge, de l'huile, de l'alcool à 90° et de l'eau.

2. Association synergique selon la revendication n°1 **caractérisée par le fait qu'**elle se présente sous la forme d'un onguent.

3. Association synergique selon les revendications précédentes **caractérisée par le fait qu'**elle arrête la chute des cheveux et favorise leur repousse.

4. Association synergique selon les revendications précédentes **caractérisée en ce qu'**elle diminue progressivement les tâches de vieillesse et les rides sur le visage.

5. Utilisation de l'association selon les revendications précédentes pour préparer un onguent destiné à supprimer l'inflammation dans les phlébites et les varices.

6. Association synergique selon les revendications précédentes **caractérisée en ce qu'**elle contient:
du miel d'acacia,
du lierre,
de l'orge
mélangés à de l'alcool à 90°
et à de l'eau , de l'huile d'olive, de tournesol ou de maïs.
